(19)

Europäisches
Patentamt

European
Patent Office

Office européen
des brevets

(11) **EP 2 558 074 B1**

## (12) EUROPEAN PATENT SPECIFICATION

(45) Date of publication and mention
of the grant of the patent:
**06.06.2018 Bulletin 2018/23**

(21) Application number: **11766711.3**

(22) Date of filing: **07.04.2011**

(51) Int Cl.:
***A61K 9/127*** (2006.01)

(86) International application number:
**PCT/US2011/031540**

(87) International publication number:
**WO 2011/127255 (13.10.2011 Gazette 2011/41)**

(54) **PREPARATION OF LIPID NANOPARTICLES**

HERSTELLUNG VON LIPIDNANOPARTIKELN

PRÉPARATION DE NANOPARTICULES DE LIPIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.04.2010 US 322054 P**

(43) Date of publication of application:
**20.02.2013 Bulletin 2013/08**

(73) Proprietor: **The Trustees of Princeton University
Princeton, NJ 08544 (US)**

(72) Inventors:
- **KUMAR, Varun
  Princeton
  New Jersey 08450 (US)**
- **PRUD'HOMME, Robert, K.
  Lawrence
  New Jersey 08648 (US)**
- **BURKE, Paul, A.
  Boston, Massachusetts 02215 (US)**
- **GINDY, Marian, E.
  West Point, Pennsylvania 19486 (US)**
- **MATHRE, David, J.
  West Point, Pennsylvania 19486 (US)**

(74) Representative: **Müller-Boré & Partner
Patentanwälte PartG mbB
Friedenheimer Brücke 21
80639 München (DE)**

(56) References cited:
WO-A1-2009/061406    WO-A1-2010/021865
US-A1- 2004 142 025    US-A1- 2005 155 541
US-A1- 2007 042 031    US-A1- 2008 299 205
US-A1- 2010 015 218

- LIU Y ET AL: "Mixing in a multi-inlet vortex mixer (MIVM) for flash nano-precipitation", CHEMICAL ENGINEERING SCIENCE, OXFORD, GB, vol. 63, no. 11, 1 June 2008 (2008-06-01), pages 2829-2842, XP022652635, ISSN: 0009-2509, DOI: 10.1016/J.CES.2007.10.020 [retrieved on 2007-10-22]
- ROHIT KARNIK ET AL: "Microfluidic Platform for Controlled Synthesis of Polymeric Nanoparticles", NANO LETTERS, vol. 8, no. 9, 10 September 2008 (2008-09-10), pages 2906-2912, XP55072920, ISSN: 1530-6984, DOI: 10.1021/nl801736q
- ANDREAS JAHN ET AL: "Microfluidic Directed Formation of Liposomes of Controlled Size", LANGMUIR, vol. 23, no. 11, 1 May 2007 (2007-05-01), pages 6289-6293, XP055036503, ISSN: 0743-7463, DOI: 10.1021/la070051a
- MARIAN E. GINDY ET AL: 'Composite Block Copolymer Stabilized Nanoparticles: Simultaneous Encapsulation of Organic Actives and Inorganic Nanostructures' LANGMUIR vol. 24, no. 1, 01 January 2008, pages 83 - 90, XP055131811 DOI: 10.1021/la702902b ISSN: 0743-7463

## Description

BACKGROUND OF THE INVENTION

Many systems for administering active substances into cells are already known. These include liposomes, nanoparticles, polymer particles, immuno- and ligand-complexes and cyclodextrins (see, Thug Transport in antimicrobial and anticancer chemotherapy. G. Papadakou Ed., CRC Press, 1995). Liposomes are typically prepared in the laboratory by sonication, detergent dialysis, ethanol injection or dilution, French press extrusion, ether infusion, and reverse phase evaporation. Liposomes with multiple bilayers are known as multilamellar lipid vesicles (MLVs). MLVs are candidates for time release drugs because the fluids entrapped between layers are only released as each membrane degrades. Liposomes with a single bilayer are known as unilamellar lipid vesicles (UV). UVs may be made small (SUVs) or large (LUVs).

Some of the methods above for liposome production impose harsh or extreme conditions which can result in the denaturation of the phospholipid raw material and encapsulated drugs. In addition, these methods are not readily scalable for mass production of large volumes of liposomes. Further, liposome formation by conventional ethanol dilution, involves the injection or dropwise addition of lipid in an aqueous buffer. The resulting liposomes are typically heterogenous in size.

Conventional liposomes are formulated to carry therapeutic agents either contained within the aqueous interior space (water-soluble drugs) or partitioned into the lipid bilayer(s) (water-insoluble drugs). Active agents which have short half-lives in the bloodstream are particularly suited to delivery via liposomes. Many anti-neoplastic agents, for example, are known to have a short half-life in the bloodstream such that their parenteral use is not feasible. However, the use of liposomes for site-specific delivery of active agents via the bloodstream is severely limited by the rapid clearance of liposomes from the blood by cells of the reticuloendothelial system.

U.S. Pat No. 5,478,860, which issued to Wheeler et al., on Dec. 26,1995 discloses microemulsion compositions for the delivery of hydrophobic compounds. Such compositions have a variety of uses. In one embodiment, the hydrophobic compounds are therapeutic agents including drugs. The patent also discloses methods for in vitro and in vivo delivery of hydrophobic compounds to cells.

PCT Publication WO01/05373 to Knopov et al., discloses techniques for preparing liposomes using an ethanol injection-type process with a static mixer that provides a turbulent environment. Therapeutic agents may then be loaded after liposome formation.

Published U.S. Application 2004/0142025, discloses techniques for forming lipid particles using a sequential stepwise dilution process. The process disclosed uses a "T"-connector or mixing chamber as mixing environment. In said mixing chamber, fluid flows meet in a narrow aperture within the "T"-connector as opposing flows at approximately 180° relative to each other. Lipid particles having optimal sizes below 200 nm are prepared when substantially equal flow rates of the flow lines are used. However, the disclosed processes tend to result in less than optimal particle sizes and less than optimal homogeneity under conditions when non-equal flow rates of the fluid lines are used. For example, US2004/0142025 restricts the variance between flow rates to less that 50%, more typically to less than about 25% and *even* more typically less than about 5%. Thus, a primary limitation of a "T"-connector or mixing chamber is the requirement of equal momenta of the fluid flows (i.e. solvent and buffer streams) to effect sufficient mixing.

Prud'homme and colleagues developed a multi-inlet vortex mixer (MIVM) to overcome limitations of the confined impinging jet (CIJ) mixer. Liu Y. et al. (2008) Chemical Engineering Science 63:2829-2842. The MIVM has been utilized in processes for preparing multicomponent composite nanoparticles. WO2009/061406. In addition, Marian E. Gindy ET AL: "Composite Block Copolymer Stabilized Nanoparticles: Simultaneous Encapsulation of Organic Actives and Inorganic Nanostructures",Langmuir, vol. 24, no. 1, 1 January 2008 (2008-01-01), pages 83-90 discloses the use of nanoparticles prepared by a flash nanoprecipitation process in a multi-inlet vortex mixer (MIVM). Despite the advances disclosed in U.S. Pat No. 5,478,860, US2004/0142025 and WO01/05373, there exists a need for improved processes for formulating and producing lipid particles, and in particular lipid particles encapsulating therapeutic agent(s) such as nucleic acid(s). The present invention fulfills these and other needs. Herein, we demonstrate a novel process to generate lipid nanoparticles which encapsulate therapeutic products, in particular nucleic acids. In particular, we disclose the use of the MIVM in this novel process.

SUMMARY OF THE INVENTION

The present invention provides a process for producing lipid nanoparticles (LNPs) encapsulating therapeutic products, said process comprising: a) providing one or more aqueous solutions in one or more reservoirs; b) providing one or more organic solutions in one or more reservoirs, wherein one or more of the organic solutions contains a lipid and wherein one or more of the aqueous solutions and/or one or more of the organic solutions includes therapeutic products; c) mixing said one or more aqueous solutions with said one or more organic solutions in a first mixing region, wherein said one or more aqueous solutions and said one or more organic solutions are introduced into a mixing chamber so as to substantially instantaneously produce a lipid nanoparticle solution containing lipid nanoparticles encapsulating therapeutic products.

In an embodiment, the present invention provides a process for producing lipid nanoparticles encapsulating therapeutic products, said process comprising: a) providing one to four aqueous solutions in separate reservoirs; b) providing one to four organic solutions in separate reservoirs, wherein one to four of the organic solutions contains a lipid and wherein one to four of the aqueous solutions and/or one to four of the organic solutions includes therapeutic products; c) mixing said one to four aqueous solutions with said one to four organic solutions in a first mixing region, wherein said one to four aqueous solutions and said one to four organic solutions are introduced into a mixing chamber so as to substantially instantaneously produce a lipid nanoparticle solution containing lipid nanoparticles encapsulating therapeutic products.

In an embodiment, the present invention provides a process for producing lipid nanoparticles encapsulating therapeutic products, said process comprising: a) providing one to three aqueous solutions in separate reservoirs; b) providing one to three organic solutions in separate reservoirs, wherein one to three of the organic solutions contains a lipid and wherein one to three of the aqueous solutions and/or one to three of the organic solutions includes therapeutic products; c) mixing said one to three aqueous solutions with said one to three organic solutions in a first mixing region, wherein said one to three aqueous solutions and said one to three organic solutions are introduced into a mixing chamber so as to substantially instantaneously produce a lipid nanoparticle solution containing lipid nanoparticles encapsulating therapeutic products.

In an embodiment, the present invention provides a process for producing lipid nanoparticles encapsulating therapeutic products, said process comprising: a) providing one to two aqueous solutions in separate reservoirs; b) providing one to two organic solutions in separate reservoirs, wherein one to two of the organic solutions contains a lipid and wherein one to two of the aqueous solutions and/or one to two of the organic solutions includes therapeutic products; c) mixing said one to two aqueous solutions with said one to two organic solutions in a first mixing region, wherein said one to two aqueous solutions and said one to two organic solutions are introduced into a mixing chamber so as to substantially instantaneously produce a lipid nanoparticle solution containing lipid nanoparticles encapsulating therapeutic products.

In an embodiment, the present invention further provides a process for producing lipid nanoparticles encapsulating therapeutic products, said process comprising: a) providing one or more aqueous solutions in one or more reservoirs; b) providing one or more organic solutions in one or more reservoirs, wherein one or more of the organic solutions contains a lipid and wherein one or more of the aqueous solutions and/or one or more of the organic solutions includes therapeutic products; c) mixing said one or more aqueous solutions with said one or more organic solutions in a first mixing region, wherein said first mixing region is a Multi-Inlet Vortex Mixer (MIVM), wherein said one or more aqueous solutions and said one or more organic solutions are introduced tangentially into a mixing chamber within the MIVM so as to substantially instantaneously produce a lipid nanoparticle solution containing lipid nanoparticles encapsulating therapeutic products.

In an embodiment, the process further comprises diluting of said lipid nanoparticle solution in an aqueous buffer immediately after mixing so as to produce a diluted lipid nanoparticle solution.

In another embodiment, the process further comprises mixing said lipid nanoparticle solution in an aqueous buffer thus introducing the lipid nanoparticle solution into a buffer reservoir containing the aqueous buffer.

In another embodiment, the buffer reservoir contains an amount of aqueous buffer substantially equal to or greater than the amount of lipid nanoparticle solution introduced thereto.

In another embodiment, the buffer reservoir is stirred.

In another embodiment, the process further comprises mixing said lipid nanoparticle solution with the aqueous buffer in a second mixing region.

In another embodiment, the process further comprises a second mixing region which is a second Multi-Inlet Vortex Mixer (MIVM) wherein the lipid nanoparticle solution and aqueous buffer are introduced tangentially into said second MIVM mixing chamber.

In another embodiment, the process further comprises a lipid nanoparticle solution that has a concentration of about 5% v/v to about 55% v/v organic solvent.

In another embodiment, the process further comprises a diluted lipid nanoparticle solution that has a concentration of less than about 25% v/v organic solvent.

In another embodiment, the process further comprises lipid nanoparticles that are less than about 150 nm in diameter.

In another embodiment, the process further comprises therapeutic products that are nucleic acids.

In another embodiment, the process further comprises nucleic acids that are siRNAs.

BRIEF DESCRIPTION OF THE DRAWINGS

FIG. 1 provides a flow diagram for a manufacturing process (100) according to one embodiment of the present invention.

FIG. 2 provides schematic representations of processes of making lipid nanoparticles according to two embodiments of the present invention.

**FIG. 3** provides schematic representation of two-stream (A, **300**) and four-stream (B, **302**) Multi-Inlet Vortex Mixers (MIVM) according to two embodiments of the present invention. Fluid streams are brought into a central mixing chamber and then expelled through a central orifice.

**FIG. 4** provides a schematic (A) and photograph (B) of MIVM mixing chamber (**330**) geometry according to one embodiment of the present environment. The main chamber has four tangential inlet streams. The outlet is at the bottom and perpendicular to the inlets.

**FIG. 5** provides schematic representations of processes and apparatus for formation of Lipid Nanoparticles (LNPs) according to three embodiments of the present invention.

**FIG. 6** shows the effect of inlet flow rate on (A) mean diameter, siRNA encapsulation, and (B) particle size distribution (PDI) of LNPs. LNPs are prepared by mixing lipid-containing organic solution (e.g., ethanol) with siRNa-containing buffer (e.g., citrate) in a MIVM. Organic and buffer solutions are mixed in a 1:1 v/v% ratio. LNP properties are measured after dilution and buffer exchange steps.

**FIG. 7** shows the effect of organic concentration on LNP diameter and encapsulation efficiency. LNPs are prepared by mixing lipid-containing organic solution (e.g. ethanol) with siRNA-containing buffer (e.g. citrate). LNP mean diameter and siRNA encapsulation are measured after dilution step.

**FIG. 8** shows the effect of organic concentration on LNP diameter and PDI. LNPs are prepared by mixing lipid-containing organic solution (e.g. tetrahydrofuran, THF) with siRNA-containing buffer (e.g. citrate). LNP mean diameter and PDI are measured after dilution step.

**FIG. 9** shows a comparison between three embodiments of the present invention; one-stage (A) and two-stage (B, C) mixing and dilution processes. LNPs are prepared by mixing lipid-containing organic solution (e.g. tetrahydrofuran, THF) with siRNA-containing buffer (e.g. citrate) at varying organic concentration. Dilution of particle suspensions after mixing is performed in accordance with each embodiment. LNP mean diameters (D) and PDI (E) are measured after dilution step.

## DETAILED DESCRIPTION OF THE INVENTION

### DEFINITIONS

Suitable therapeutic products (otherwise referred to as "therapeutic agents") include, but are not limited to, a protein, a nucleic acid, an antisense nucleic acid, a ribozyme, tRNA, snRNA, siRNA (small interfering RNA), miRNA, shRNA, ncRNA, pre-condensed DNA, an aptamer and an antigen. In certain preferred aspects, the therapeutic product(s) is/are nucleic acid(s) and/or oligonucleotides. In certain more preferred aspects, the therapeutic product(s) is/are siRNA(s).

The term "nucleic acid" refers to a polymer containing at least two nucleotides. "Nucleotides" contain a sugar deoxyribose (DNA) or ribose (RNA), a base, and a phosphate group. Nucleotides are linked together through the phosphate groups (although synthetic nucleic acids may be prepared using nucleotide linkers other than phosphate groups). "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides.

DNA may be in the form of antisense, plasmid DNA, parts of a plasmid DNA, pre-condensed DNA, product of a polymerase chain reaction (PCR), vectors (PI, PAC, BAC, YAC, artificial chromosomes), expression cassettes, cbimeric sequences, chromosomal DNA, or derivatives of these groups.

"Antisense" is a polynucleotide that interferes with the function of DNA and/or RNA. This may result in suppression of expression. Natural nucleic acids have a phosphate backbone, artificial nucleic acids may contain other types of backbones and bases. These include PNAs (peptide nucleic acids), phosphothioates, and other variants of the phosphate backbone of native nucleic acids.

RNA may be in the form of oligonucleotide RNA, tRNA (transfer RNA), snRNA (small nuclear RNA), rRNA (ribosomal RNA), mRNA (messenger RNA), antisense RNA, siRNA (small interfering RNA), miRNA, shRNA (shorthairpin RNA), ncRNA (non-coding RNA), aptamers, ribozymes, cbimeric sequences, or derivatives of these groups.

"siRNA" directs the sequence-specific silencing of mRNA through a process known as RNA interference (RNAi). The process occurs in a wide variety of organisms, including mammals and other vertebrates. Methods for preparing and administering siRNA and their use for specifically inactivating gene function are known. siRNA includes modified and unmodified siRNA. Examples and a further discription of siRNA, including modification to siRNAs can be found in WO2009/126933, which is hereby incorporated by reference.

In addition, DNA and RNA may be single, double, triple, or quadruple stranded.

As used herein, the term "aqueous solution" refers to a composition comprising in whole, or in part, water.

As used herein, the term "organic solution" refers to a composition comprising in whole, or in part, an organic solvent.

As used herein, the term "mixing region" optionally is any "mixer" known in the art. Particular mixers known in the art include a confined impinging jet (CIJ) mixer or a multi-inlet vortex mixer (MIVM).

As used herein, a "mixer" refers to a device with three or more inlets meeting in a central mixing chamber designed to enhance mixing, and a single outlet. In another embodiment, a "mixer" refers to a device with four inlets, meeting in a central mixing chamber, and a single outlet. In another embodiment, the "mixer" is a MIVM.

The term "lipid" refers to a group of organic compounds that are esters of fatty acids and are characterized by being insoluble in water but soluble in many organic solvents. They are usually divided in at least three classes: (1) "simple lipids" which include fats and oils as well as waxes; (2) "compound lipids" which include phospholipids and glycolipids; (3) "derived lipids" such as steroids.

The term "amphipathic lipid" refers, in part, to any suitable material wherein the hydrophobic portion of the lipid material orients into a hydrophobic phase, while a hydrophilic portion orients toward the aqueous phase. Amphipathic lipids are usually the major component of lipid nanoparticles. Hydrophilic characteristics derive from the presence of polar or charged groups such as carbohydrates, phosphate, carboxylic, sulfate, amino, sulfhydryl, amine, hydroxy and other like groups. Hydrophobicity can be conferred by the inclusion of apolar groups that include, but are not limited to, long chain saturated and unsaturated aliphatic hydrocarbon groups and such groups substituted by one or more aromatic, cycloaliphatic or heterocyclic group(s). Examples of amphipathic compounds include, but are not limited to, phospholipids, aminolipids and sphingolipids. Representative examples of phospholipids include, but are not limited to, phosphatidylcholine, phosphatidylethanolamine, phosphatidylserine, phosphatidylinositol, phosphatidic acid, palmitoyloleryl phosphatidylcholine, lysophosphatidylcholine, lysophosphatidylethanolamine, dipalmitoylphosphatidylcholine, dioleoylphospbatidylcholine, dstearoylphosphatidylcholine or dilinoleoylphosphatidylcholine. Other compounds lacking in phosphorus, such as sphingolipid, glycosphingolipid families, diacylglycerols and S-acyloxyacids, are also within the group designated as amphipathic lipids. Additionally, the amphipathic lipid described above can be mixed with other lipids including triglycerides and sterols.

The term "neutral lipid" refers to any of a number of lipid species that exist either in an uncharged or neutral zwitterionic form at a selected pH. At physiological pH, such lipids include, for example, diacylphosphatidylcholine, diacylphosphatidyletbanolamine, ceramide, sphingomyelin, cephalin, cholesterol, cerebrosides and diacylglycerols.

The term "noncationic lipid" refers to any neutral lipid as described above as well as anionic lipids. Useful noncationic lipids include, for example, distearoylphosphatidylcholine (DSPC), dioleoylphosphatidylcholine (DOPC), dipalmitoylphosphatidylcholine (OPEC), dioleoylphospbatidylglycerol (DOPG), dipahnitoylphosphatidylglycerol (DPPG), dioleoyl-phosphatidylethanolamine (DOPE), palmitoyloleoylphosphatidylcholine (POPC), palmitoylolmyl-phosphatidylethanolamine (POPE) and dioleoyl-phosphatidylethanolamine 4-(4-maleimidomethyl)cyelohexane-1-carboxylate (DOPE-teal), dipahnitoyl phosphatidyl ethanolamine (DPPE), dimyristoylphosphoetbanolamine (DMPE), distearoyl-phosphatidylethanolamine (DSPE), 16-O-monomethyl PE, 16-0dimethyl PE, 18-1-trans PE, 1-stearoyl-2-oleoyl-phosphatidyethanolamine (SOPS), and 1,2-dielaidoyl-sn-glycero-3-phophoethanolamine (transDOPE).

The term "anionic lipid" refers to any lipid that is negatively charged at physiological pH. These lipids include, but are not limited to, phosphatidylglycerol, cardiolipin, diacylphosphatidylserine, diacylphosphatidic acid, N-dodecanoyl phosphatidylethanolamines, N-succinyl phosphatidylethanolamines, N-glutarylphosphatidylethanolamines, lysylphosphatidylglycerols, palmitoyloleyolphosphatidylglyeerol (POPG), and other anionic modifying groups joined to neutral lipids.

The term "cationic lipid" refers to any of a number of lipid species which carry a net positive charge at a selective pH, such as physiological pH. Such lipids include, but are not limited to, N,N-dioleyl-N,N-dimethylammonium chloride ("DODAC"); N-(2,3dioleyloxy)propyl)-N,N,Ntrimethylammonium chloride ("DOTMA"); N,NdistearylN,N-dimethylammonium bromide ("DDAB"); N-(2,3dioleoyloxy)propyl)-N,N,N-trimethylamntonium chloride ("DODAP"); 3-(N-(N,N-dimethylaminoethane)-carbamoyl)cholesterol ("DC-Chol") and N-(1,2-dimyristyloxyprop-3-yl)-N,N-dimethyl-N-hydmxyethyl ammonium bromide ("DMRIE"). Additionally, a number of commercial preparations of cationic lipids are available which can be used in the present invention. These include, for example, LIPOFECTIN® (commercially available cationic lipid nanoparticles comprising DOTMA and 1,2dioleoyl-sn-3-phosphoethanolamine ("DOPE"), from GIBCOBRL, Grand Island, N.Y, USA); LIPOFECTAMINE® (commercially available cationic lipid nanoparticles comprising N-(1-(2,3dioleyloxy)propyl)N-(2-(sperminecarboxamido)ethyl)-N,N-dimethylammonium trifluoroacetate ("DOSPA') and ("DOPE"), from (3IBCOBRL); and TRANSFECTAM® (commercially available cationic lipids comprising diocmdecylamidoglycyl carboxyspermine ("DOGS") in ethanol from Promega Corp., Madison, Wis., USA). The following lipids are cationic and have a positive charge at below physiological pH: DODAP, DODMA, DMDMA, 1,2-DiLinoleyloxy-N,N-dimethylaminopropane (DLinDMA), 4-(2,2-diocta-9,12-dienyl-[1,3]dioxolan-4-ylmethyl)-dimethylamine, DLinKDMA (WO 2009/132131 A1), DLin-K-C2-DMA (WO2010/042877), DLin-M-C3-DMA (WO2010/146740 and/or WO2010/105209), 2-{4-[(3β)-cholest-5-en-3-yloxy]butoxy}-N,N-dimethyl-3-[(9Z,12Z)-octadeca-9,12-dienlyloxyl]propan-1-amine) (CLinDMA), and the like.

In addition to cationic and non-cationic lipids, the lipid nanoparticles of the present invention may comprise bilayer stabilizing component (BSC) such as an ATTA-lipid or a PEG-lipid, such as PEG coupled to dialkyloxypropyls (PEG-DAA) as described in, e.g., WO 05/026372, PEG coupled to diacylglycerol (PEG-DAG) as described in, e.g., U.S. Patent Publication Nos. 20030077829 and 2005008689, PEG coupled to dimyristoylglecerol (PEG-DMG) as described in, e.g., Abrams et. al., Molecular Therapy 2010, 18(1), 171, PEG coupled to phosphatidylethanolamine (PE) (PEG-PE), or PEG conjugated to 1,2-Di-O-hexadecyl-sn-glyceride (PEG-DSG), or a mixture thereof (see, U.S. Pat. No. 5,885,613). In one preferred embodiment, the BSC is a conjugated lipid that inhibits aggregation of the lipid nanoparticle.

In certain aspects, the cationic lipid typically comprises from about 2% to about 70%, from about 5% to about 50%, from about 10% to about 45%, from about 20% to about 40%, or from about 30% to about 40% of the total lipid present in said particle. The non-cationic lipid typically comprises from about 5% to about 90%, from about 10% to about 85%, from about 20% to about 80%, from about 30% to about 70%, from about 40% to about 60% or about 48% of the total lipid present in said particle. The PEG-lipid conjugate typically comprises from about 0.5% to about 20%, from about 1.5% to about 18%, from about 4% to about 15%, from about 5% to about 12%, or about 2% of the total lipid present in said particle. The nucleic acid-lipid particles of the present invention may further comprise cholesterol. If present, the cholesterol typically comprises from about 0% to about 10%, about 2% to about 10%, about 10% to about 60%, from about 12% to about 58%, from about 20% to about 55%, or about 48% of the total lipid present in said particle. It will be readily apparent to one of skill in the art that the proportions of the components of the nucleic acid-lipid particles may be varied.

In some embodiments, the nucleic acid to lipid ratios (mass/mass ratios) in a formed nucleic acid-lipid particle will range from about 0.01 to about 0.3. The ratio of the starting materials also falls within this range

"Lipid Nanoparticle" refers to any lipid composition that can be used to deliver a therapeutic product, preferably siRNAs or an siRNA, including, but not limited to, liposomes or vesicles, wherein an aqueous volume is encapsulated by amphipathic lipid bilayers (*i.e.* single; unilamellar or multiple; multilamellar), or wherein the lipids coat an interior comprising a therapeutic product, or lipid aggregates or micelles, wherein the lipid encapsulated therapeutic product is contained within a relatively disordered lipid mixture.

As used herein, "lipid encapsulated" can refer to a lipid formulation which provides a therapeutic product with full encapsulation, partial encapsulation, or both.

As used herein, the term "LNP" refers to a lipid nanoparticle.

## GENERAL

The present invention provides processes for making lipid nanoparticles. The processes can be used to make lipid nanoparticles possessing a wide range of lipid components including, but not limited to, cationic lipids, anionic lipids, neutral lipids, polyethylene glycol (PEG) lipids, hydrophilic polymer lipids, fusogenic lipids and sterols. Hydrophobic actives can be incorporated into the organic solvent (e.g., ethanol) with the lipid, and nucleic acid and hydrophilic actives can be added to an aqueous component. In certain aspects, the processes of the present invention can be used in preparing microemulsions where a lipid monolayer surrounds an oil-based core. In certain aspects, the processes and apparatus are used in preparing lipid nanoparticles, wherein a therapeutic agent is encapsulated within a lipid nanoparticle coincident with lipid nanoparticle formation.

## PROCESS OF MAKING

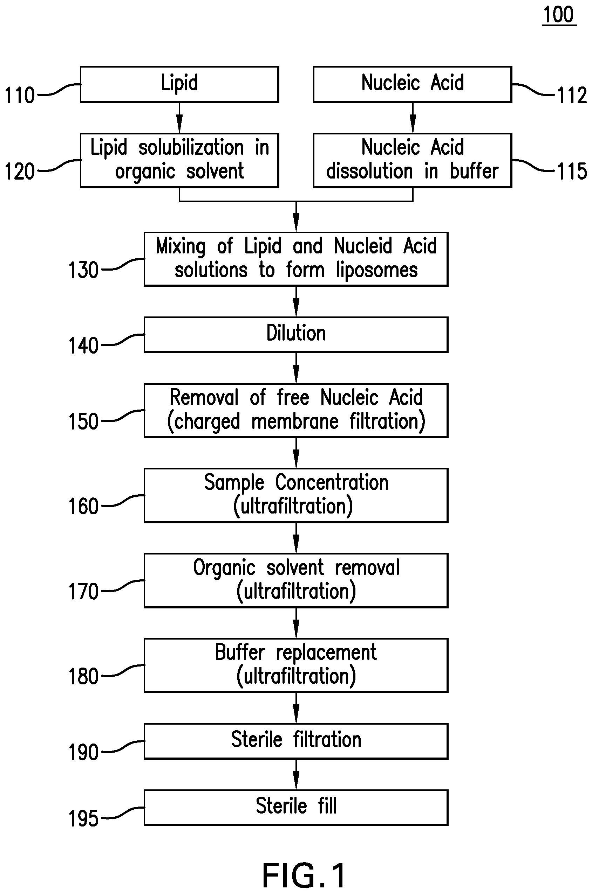

FIG. 1 is an example of a representative flow chart **100** of a method of the present invention. This flow chart is merely an illustration and should not limit the scope of the claims herein. One of ordinary skill in the art will recognize other variations, modifications, and alternatives.

In one aspect, the present method provides a lipid solution **110** such as a clinical grade lipid synthesized under Good Manufacturing Practice (GMP), which is thereafter solubilized in an organic solution **120** (e.g., ethanol). Similarly, a therapeutic product, e.g., a therapeutic active agent such as nucleic acid **112** or other agent, is prepared under GMP. Thereafter, a therapeutic agent solution (e.g., nucleic acids) **115** containing a buffer (e.g., citrate) is mixed with a lipid solution **120** solubilized in an organic solution to form a lipid nanoparticle formulation **130** (also referred to herein as "lipid nanoparticle suspension" or "lipid nanoparticle solution"). The therapeutic agent is entrapped in the lipid nanoparticle substantially coincident with formation of the lipid nanoparticle. Typically, an electrostatic interaction between the negatively charged nucleic acid and positively charged cationic lipid brings about encapsulation. If a titratable cationic lipid is used, for example, poor nucleic acid encapsulation efficiencies may be achieved at higher pH approaching or exceeding the cationic lipids pKa. Those of skill in the art will realize, however, that the processes and apparatus of the present invention are equally applicable to active entrapment or loading of the lipid nanoparticles after formation of the lipid nanoparticle. In certain aspects, the lipid nanoparticle solution is substantially immediately mixed with a buffer solution **140** to dilute the nanoparticle solution (i.e., suspension of lipid nanoparticles).

According to the processes and systems of the present invention, the action of continuously introducing lipid and buffer solutions into a mixing environment, such as in a mixing chamber, causes a continuous dilution of the lipid solution with the buffer solution, thereby producing a lipid nanoparticle substantially instantaneously upon mixing. Immediately diluting the lipid nanoparticle suspension, e.g., mixing the lipid nanoparticle suspension with buffer, helps prevent lipid nanoparticle particle sizes from increasing as would typically be the case if the lipid nanoparticle suspension is allowed to sit for an extended period of time, e.g., minutes or hours. Also, immediate dilution further enhances lipid nanoparticle homogeneity especially where siRNA is the encapsulated therapeutic agent. As used herein, the phrase "continuously diluting a lipid solution with a buffer solution" (and variations) generally means that the lipid solution is diluted sufficiently rapidly in a hydration process with sufficient force to effectuate lipid nanoparticle generation.

In the processes of the present invention, the organic lipid solution typically includes an organic solvent, such as a lower alcohol. As mentioned above, in one aspect, the lipid nanoparticles are immediately diluted **140** with a buffer (e.g., citrate) to increase nucleic acid (e.g., plasmid or siRNA) entrapment and maintain particle size. Such dilution may be by way of immediate introduction of the lipid nanoparticle solution into a controlled amount of buffer solution, or by mixing the lipid nanoparticle solution with a controlled flow rate of buffer in a second mixing region. Dilution can also be effected coincident with lipid nanoparticle formation **130** at initial introduction of organic lipid and buffer solutions into a mixing environment. In one embodiment, organic lipid and buffer solutions are introduced into a mixing environment at substantially non-equal flow-rates such that resulting lipid nanoparticle solution contains a volumetric excess of dilution buffer.

Before sample concentration **160,** free therapeutic agent (e.g., nucleic acid) is removed by using, for example, an anion exchange cartridge **150.** Further, by using an ultrafiltration step **170** to remove the organic solution, the sample is concentrated (e.g., to about 0.9 mg/mL nucleic acid), the organic solution (e.g. alcohol) is removed, and the buffer is replaced with a substitute buffer (e.g., with a saline buffer) **180.** Thereafter, the sample is filtered **190** and filled in vials **195.** The process will now be discussed in more detail herein below using the steps as set forth in FIG. 1.

Lipid Solubilization and Therapeutic Agent Dissolution

In one embodiment, the lipid nanoparticle produced according to the processes of the present invention include nucleic acid lipid nanoparticle (i.e., LNP) formulations. Those of skill in the art will appreciate that the following description is for illustration purposes only. The processes of the present invention are applicable to a wide range of lipid nanoparticle types and sizes. These lipid nanoparticles include, but are not limited to, single bilayer lipid vesicles known as unilamellar lipid vesicles which can be made small (SUVs) or large (LUVs), as well as multilamellar lipid vesicles (MLVs). Further vesicles include, micelles, lipid nucleic acid particles, virosomes, and the like. Those of skill in the art will know of other lipid vesicles for which the processes and apparatus of the present invention will be suitable.

The preferred size for lipid nanoparticles made in accordance with the present processes and apparatus are between about 25-200 nm in diameter. In certain aspects, the lipid nanoparticle preparation has a size distribution in which the mean size (e.g., diameter) is about 70 nm to about 200 nm, and more typically the mean size is about 100 nm or less.

In certain aspects, the lipid nanoparticle formulation (e.g., LNP formulation) of the present invention includes four lipid components; a phospholipid; cholesterol; a PEG-lipid; and a cationic lipid. In one aspect, the phospholipid is DSPC, the PEG-lipid is PEG-S-DMG and the cationic lipid is CLinDMA or DLinDMA. In one aspect, the molar composition is about 60:38:2 CLinDMA:choesterol:PEG-DMG. In another aspect, the LNP formulation is 40:48:10:2 DLinDMA:cholesterol:DSPC:PEG-DMG. In certain embodiments, the organic solvent concentration wherein the lipids are solubilized is about 45% v/v to about 100% v/v. In certain aspects, the organic solvent is a lower alcohol. Suitable lower alcohols include, but are not limited to, methanol, ethanol, propanol, butane, pentanol, their isomers and combinations thereof. In one embodiment, the solvent is ethanol with a volume of about 50-90% v/v. In one aspect, the lipids occupy a volume of about 1 mL/g to about 5 mL/g.

The lipids are solubilized **120** using for example, an overhead stirrer at a suitable temperature. In one aspect, the total lipid concentration of the solution is about 15. mg/mL (6.8 mg/mL for LNP formulation). In certain aspects, the therapeutic agent (e.g., nucleic acid) is included in an aqueous solution (e.g., buffer) and is diluted to a final concentration. In one aspect, for example, the final concentration is about 0.8 mg/mL in citrate buffer, with a pH of about 4-6. In this instance, the volume of the nucleic acid solution is the same as the alcohol-lipid solution. It should be appreciated that the buffer solution need not be acidic when using the direct dilution approaches of the present invention, e.g, the pH of the buffer solution can be 7.0 or higher. In one embodiment, the preparation of the therapeutic agent (e.g., nucleic acid) solutions performed in a jacketed stainless steel vessel with an overhead mixer. The sample does not need to be heated to be prepared, although in certain instances it is at the same temperature as the lipid solution prior to lipid nanoparticle formation.

Lipid Nanoparticle Formation

After the solutions, e.g., lipid solution **120** and aqueous therapeutic agent (e.g., nucleic acid) solution **115,** have been prepared, they are mixed together **130** using, for example, a peristaltic pump mixer or a pulseless gear pump. In one aspect, the solutions are pumped at substantially equal flow rates into a mixing environment. In preferred aspects, the mixing environment includes a Multi-Inlet Vortex Mixer (MIVM) or mixing chamber. In one aspect, the solutions are pumped at substantially equal flow rates into the MIVM mixing environment. In another aspect, the solutions are pumped at substantially unequal flow rates. Upon meeting and mixing of the solution flows in the mixing environment, lipid nanoparticles are substantially instantaneously formed. Lipid nanoparticles are formed when an organic solution including dissolved lipid and an aqueous solution (e.g., buffer) are simultaneously and continuously mixed. Advantageously, by mixing the aqueous solution with the organic lipid solution, the organic lipid solution undergoes a continuous, sequential stepwise dilution to substantially instantaneously produce a lipid nanoparticle solution. The pump mechanism(s) can be configured to provide equivalent or different flow rates of the lipid and aqueous solutions into the mixing environment.

Advantageously, the apparatus for mixing of lipid and the aqueous solutions taught herein provides for formation of lipid nanoparticles under conditions where the composition of buffer and organic solutions can be changed over a wide range, without loss of mixing efficiency. Contrary to mixing in "T"-connector or mixing chamber, where substantially equal momenta (i.e. flow rates) of the fluid flows are required to effect sufficient mixing, in the MIVM momentum (i.e. flow rate) from each stream contributes independently to drive micromixing in the mixing chamber. Therefore, it is possible to have one or more streams at high volumetric flow rate and another stream at a lower flow rate and still achieve good micromixing. In this instance, the ratio of organic to buffer solutions at initial mixing can be advantageously manipulated to effect better control over lipid particle properties (e.g. particle size and particle size stability).

Also advantageously, the processes and apparatus for mixing of the lipid solution and the aqueous solutions taught herein provides for encapsulation of therapeutic agent in the formed lipid nanoparticle substantially coincident with lipid nanoparticle formation with an encapsulation efficiency of up to about 90%. Further processing steps as discussed herein can be used to further refine the encapsulation efficiency and concentration if desired.

In one embodiment, lipid nanoparticles form when lipids dissolved in an organic solvent (e.g., ethanol) are diluted in a stepwise manner by mixing with an aqueous solution (e.g., buffer). This controlled stepwise dilution is achieved by mixing the aqueous and lipid streams together in a confined volume, such as in a MIVM, and thereafter diluting in a buffer solution. The resultant lipid, solvent and solute concentrations can be kept constant throughout the nanoparticle formation process if desired. In one aspect, lipid nanoparticles are formed having a mean diameter of less than about 150 nm, e.g., about 100 nm or less, which advantageously do not require further size reduction by high-energy processes such as membrane extrusion, sonication or microfluidization.

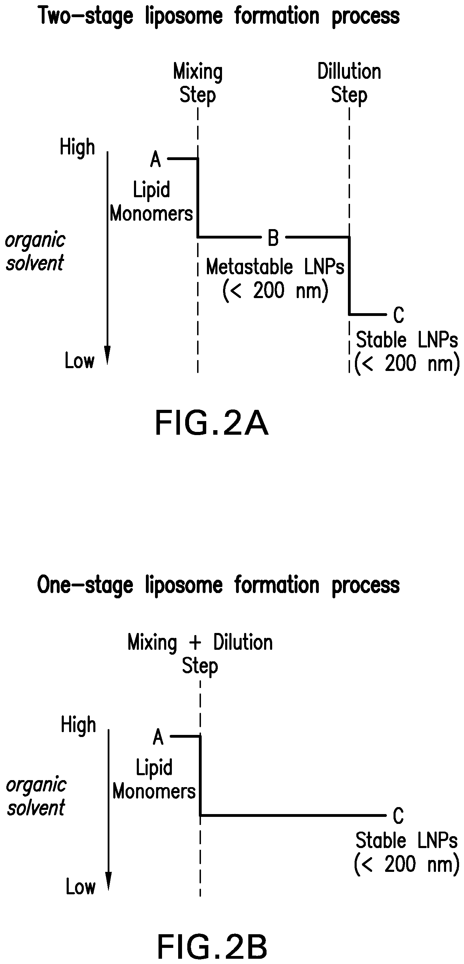

One embodiment of the inventive process is shown in FIG. 2. In one aspect, using the processes and apparatus of the present invention, a lipid nanoparticle solution is prepared by a two-stage step-wise dilution. For example, in the first stepwise dilution, lipid nanoparticles are formed in a high organic solvent (e.g., ethanol) environment (e.g., about 35% v/v to about 55% v/v organic solvent). These lipid nanoparticles can then be stabilized in a second (e.g. dilution) step by lowering the organic solvent (e.g., ethanol) concentration to less than or equal to about 25% v/v such as about 17% v/v to about 25% v/v, in a stepwise manner. Such dilution may be by way of immediate introduction of the lipid nanoparticle solution into a controlled amount of buffer solution, or by mixing the lipid nanoparticle solution with a controlled flow rate of buffer in a second mixing region. In certain aspects, with therapeutic agent present in the aqueous solution, or in the lipid solution, the therapeutic agent is encapsulated coincident with lipid nanoparticle formation.

As shown in FIG. 2A, in one embodiment, lipids are initially dissolved in an organic solvent (e.g., ethanol) environment of about 70% v/v to about 100% v/v, more typically about 65% v/v to about 90% v/v, and most typically about 80% v/v to about 100% v/v (A). Next, the lipid solution is diluted stepwise by mixing with an aqueous solution resulting in the formation of lipid nanoparticles at an organic solvent (e.g., ethanol) concentration of about 35% v/v to about 55% v/v, more typically about 33% v/v to about 45% v/v, and most typically about 40% v/v to about 50% v/v (B). By mixing the aqueous solution with the organic lipid solution, the organic lipid solution undergoes a continuous stepwise dilution to produce a lipid nanoparticle. Further, lipid nanoparticles such as LNPs can be further stabilized by an additional stepwise dilution of the nanoparticles to an alcohol concentration of less than or equal to about 25%, preferably between about 19-25% (C). In certain aspects, the additional sequential dilution (C) is performed substantially immediately after formation of the lipid nanoparticles. For example, it is advantageous that less than 1 minute elapse between lipid nanoparticle solution formation and dilution (C), more advantageously less than 10 seconds and even more advantageously less than a second or two.

In another embodiment, using the processes and apparatus of the present invention, a lipid nanoparticle solution is prepared by a one-stage step-wise process in which lipid nanoparticle formation occur coincidentally with dilution. In this instance, shown in FIG. 2B, lipids are initially dissolved in an organic solvent (e.g., ethanol) environment of about 70% v/v to about 100% v/v, more typically about 65% v/v to about 90% v/v, and most typically about 80% v/v to about 100% v/v (A). Next, the lipid solution is diluted stepwise by mixing with an aqueous solution resulting in the formation of nanoparticles at an organic solvent (e.g., ethanol) concentration of about 5% v/v to about 35% v/v, more typically about 15% v/v to about 30% v/v, and most typically about 10% v/v to about 25% v/v (C). By mixing the organic lipid solution with a volumetric excess of aqueous solution (i.e., non-equal flow rates of organic solution and aqueous solution fluid flows), the organic lipid solution undergoes a stepwise dilution to produce a lipid nanoparticle, while simultaneously being diluted.

In one aspect, the lipid nanoparticles are formed at a rate of 40 to about 400 mL/min After the mixing step **130,** the lipid concentration is about 1-12 mg/mL and the therapeutic agent (e.g., nucleic acid) concentration is about 0.05-1 mg/mL. In certain preferred aspects, the lipid concentration is about 3.4 mg/ml, and the therapeutic agent (e.g., nucleic acid) concentration is about 0.8 mg/mL to give a lipid-nucleic acid ratio of about 4. The buffer concentration is about 1-25 mM and the organic solvent (e.g., alcohol) concentration is about 5% v/v to about 95% v/v. In preferred aspects, the buffer concentration is about 25 mM and the organic solvent (e.g., alcohol) concentration is about 20% v/v to about 50% v/v.

## Lipid Nanoparticle Dilution

Turning back to FIG. 1, after the nanoparticle formation step **130,** the degree of therapeutic agent (e.g., nucleic acid) encapsulation is enhanced and particle size maintained, and even reduced, by immediate diluting **140** the lipid nanoparticle solution prior to removal of free nucleic acid. For example, prior to dilution step **140,** if the therapeutic agent entrapment is at about 30-60%, it can be increased to about 80-90% following dilution step **140.** For instance, in step **140,** the lipid nanoparticle formulation is diluted to about 10% v/v to about 40% v/v, preferably about 20% alcohol, by mixing with an aqueous solution such as a buffer (e.g., 1:1 with 20 mM citrate buffer, 300 mM NaCl, pH 6.0). The diluted sample is then optionally allowed to incubate at room temperature.

Dilution may be effected by way of immediate introduction of the lipid nanoparticle solution into a controlled amount of buffer solution, or by mixing the lipid nanoparticle solution with a controlled flow rate of buffer in a second mixing region. Alternatively, dilution can be effected coincident with lipid nanoparticle formation **130,** upon initial introduction of organic lipid and buffer solutions into the mixing environment. In one embodiment, organic lipid and buffer solutions are introduced into a mixing environment at substantially non-equal flow-rates such that the resulting lipid nanoparticle solution contains a volumetric excess of dilution buffer. The diluted sample is then optionally allowed to incubate at room temperature.

## Removal of Free Therapeutic Agent

After immediate dilution **140,** about 70-80% or more of the therapeutic agent (e.g., nucleic acid) is entrapped within the lipid nanoparticle (e.g., LNP) and the free therapeutic agent can be removed from the formulation **150.** In certain aspects, anion exchange chromatography is used. Advantageously, the use of an anion exchange resin results in a high dynamic nucleic acid removal capacity, is capable of single use, may be presterilized and validated, and is fully scaleable. In addition, the method results in removal of free therapeutic agent (e.g., nucleic acid). The volume of sample after chromatography is unchanged, and the therapeutic agent (e.g., nucleic acid) and lipid concentrations are about 0.3 and 1.7 mg/mL, respectively. At this point, the sample can be assayed for encapsulated therapeutic agent.

## Sample Concentration

In certain instances, the lipid nanoparticle solution is optionally concentrated about 5-50 fold, preferably 10-20 fold, using for example, ultrafiltration **160** (e.g., tangential flow dialysis). In one embodiment, the sample is transferred to a feed reservoir of an ultrafiltration system and the buffer is removed. The buffer can be removed using various processes, such as by ultrafiltration. In one aspect, buffer is removed using cartridges packed with polysulfone hollow fibers, for example, having internal diameters of about 0.5 mm to about 1.0 mm and a 30,000 nominal molecular weight cut-off (NMWC). Hollow fibers with about a 1,000 MWCO to about a 750,000 MWCO may also be used. The lipid nanoparticles are retained within the hollow fibers; and recirculated while the solvent and small molecules are removed from the formulation by passing through the pores of the hollow fibers. In this procedure, the filtrate is known as the permeate solution. On completion of the concentration step, the therapeutic agent (e.g., nucleic acid) and lipid concentrations can increase to about 2 and 60 mg/mL, respectively. In one embodiment, the organic solvent (e.g., alcohol) concentration remains unchanged, but the organic solvent:lipid ratio decreases about 50 fold.

## Organic Solvent (e.g., alcohol) Removal

In one embodiment, the concentrated formulation is diafiltered against about 5-20 volumes, preferably about 10 volumes, of aqueous solution (e.g., citrate buffer pH 4.0 (25 mM citrate, 100 mM NaCl) to remove the alcohol **170.** A neutral buffer or a sugar-based buffer may also be used. The organic solvent (e.g., alcohol) concentration at the completion of step 170 is less than about 1%. Lipid and therapeutic agent (e.g., nucleic acid) concentrations remain unchanged and the level of therapeutic agent entrapment also remains constant.

## Buffer Replacement

After the organic solvent (e.g., alcohol) has been removed, the aqueous solution (e.g., buffer) is then replaced by diafiltration against another buffer **180** (e.g., against 10 volumes of saline 150 mM NaCl with 10 mM HEPES or Phosphate pH 7.4). Any of a variety of buffers may be used, e.g., neutral, sugar-based, etc. Typically, the ratio of concentrations of lipid to therapeutic agent (e.g., nucleic acid) remain unchanged and the level of nucleic acid entrapment is about constant. In certain instances, sample yield can be improved by rinsing the cartridge with buffer at about 10% volume of the concentrated sample. In certain aspects, this rinse is then added to the concentrated sample.

Sterile Filtration

In certain preferred embodiments, sterile filtration **190** of the sample at lipid concentrations of about 12-120 mg/mL can optionally be performed. In certain aspects, filtration is conducted at pressures below about 40 psi, using a capsule filter and a pressurized dispensing vessel with a heating jacket. Heating the sample slightly can improve the ease of filtration.

Sterile Fill

The sterile fill step **195** is performed using similar processes as for conventional liposomal formulations. The processes of the present invention result in about 50-60% of the input therapeutic agent (e.g., nucleic acid) in the final product. In certain aspects, the therapeutic agent to lipid ratio of the final product is approximately 0.01 to 0.2.

THERAPEUTIC PRODUCTS

The lipid-based drug formulations and compositions of the present invention are useful for the systemic or local delivery of therapeutic products and are also useful in diagnostic assays.

As described above, therapeutic product is preferably incorporated into the lipid nanoparticle during formation of the nanoparticle, In one embodiment, hydrophobic actives can be incorporated into the organic solvent with the lipid, while nucleic acid and hydrophilic therapeutic products can be added to the aqueous component. In certain instances, the therapeutic products includes one of a protein, a nucleic acid, an antisense nucleic acid, ribozymes, tRNA, snRNA, siRNA, miRNA sbRNA, mRNA, pre-condensed DNA, an aptamer, an antigen and combinations thereof. In preferred aspects, the therapeutic product is nucleic acid. In a more preferred aspect, the therapeutic product is a siRNA.

In certain aspects, therapeutic product is incorporated into the organic lipid component.

In another embodiment, the lipid nanoparticles of the present invention can be loaded with one or more therapeutic products after formation of the nanoparticle. In certain aspects, the therapeutic products which are administered using the present invention can be any of a variety of drugs which are selected to be an appropriate treatment for the disease to be treated. Typically, the drug is an siRNA.

APPARATUS

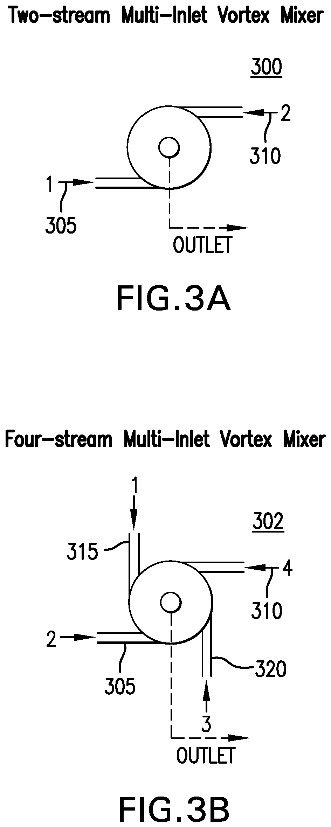

In one embodiment, the present invention provides systems and apparatus for carrying out the processes of the present invention. FIGS. 3A and 3B show examples of an apparatus **300** and apparatus **302,** respectively, according to two embodiments of the present invention. These schematics are merely illustrations and should not limit the scope of the claims herein. One of ordinary skill in the art will recognize other variations, modifications, and alternatives.

As shown, apparatus **300** includes two reservoirs, an aqueous solution reservoir **305** and an organic solution reservoir **310,** for holding aqueous solution and organic solution, respectively. Apparatus **302** includes four reservoirs, including an aqueous solution reservoir **305** and an organic solution reservoir **310,** for holding aqueous solution and organic solution, respectively. The third and fourth reservoirs **315** and **320** are used for holding either aqueous solution, or organic solution, or a combination thereof.

In certain aspects, the lipid nanoparticle formulations are prepared rapidly, at low pressure (e.g., <10 psi) and the apparatus and processes of the present invention are fully scaleable (e.g., 0.5 mL-5000 L). At a 1-L scale, lipid nanoparticles are formed at about 0.4-1.7 L/min. In certain preferred aspects, the apparatus does not use static mixers nor specialized extrusion equipment.

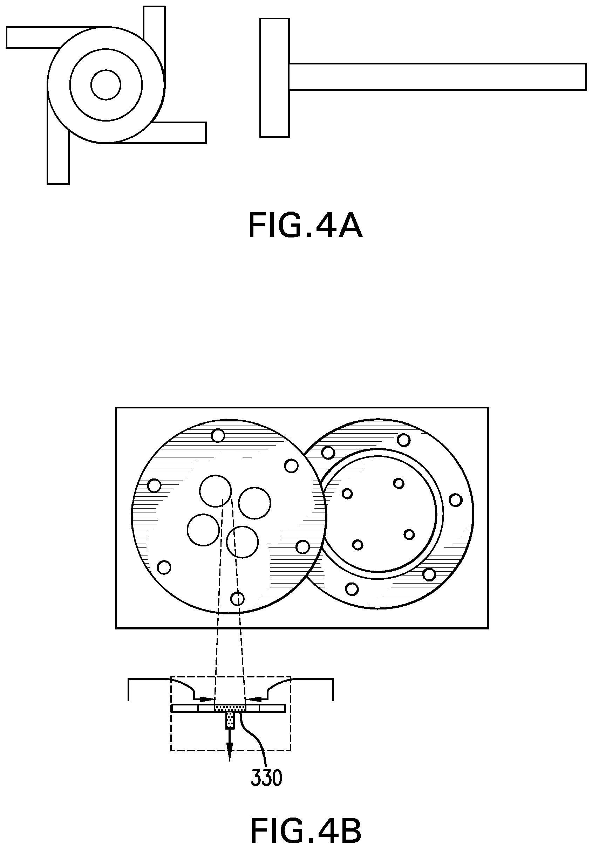

FIG. 4 shows a Multi-Inlet Vortex Mixer (MIVM) according to one embodiment. The mixing chamber **330** includes, in one embodiment, a mixing chamber, having optional hose barbs, wherein fluid lines impact each other tangentially (and not at 180° or angles thereabout, as per "T"-connector of Impinging Jets-type mixing geometries). In certain aspects, lipid nanoparticles of well defined and reproducible mean diameters are prepared using substantially equal flow rates of the flow lines. In other aspects, lipid nanoparticles of well defined and reproducible mean diameters are prepared using substantially non-equal flow rates of the fluid lines. Examples of flow rates are shown and discussed in more detail in the Example section (below).

In comparison with prior systems, the present invention provides a number of significant improvements. Advantageously, the apparatus for mixing of lipid and the aqueous solutions taught herein provides for formation of lipid nanoparticles under conditions where the concentration of buffer and organic solutions can be changed over a wide range, without loss of mixing efficiency. Contrary to mixing in "T"-connector or mixing chamber, where substantially equal momenta (i.e. flow rates) of the fluid flows are required to effect sufficient mixing, in the MIVM momentum (i.e. flow rate) from each stream contributes independently to drive micromixing in the mixing chamber. Therefore, it is possible to have one or more streams at high volumetric flow rate and another stream at a lower flow rate and still achieve good micromixing. In this instance, the ratio of organic to buffer solutions at initial mixing can be advantageously manipulated to effect better control over lipid particle properties (e.g. particle size, size stability, nucleic acid encapsulation, lipid rearrangement, etc.).

For example, the present invention advantageously permits the formation of LNPs at solvent concentrations as low as 5% v/v, more typically 10 % v/v, and even more typically 25% v/v in a single mixing step. In this capacity, therapeutic agent (e.g., nucleic acid) encapsulation is enhanced and particle size maintained, and even reduced, relative to alternative dilution strategies (e.g. dilution **140** of the lipid nanoparticle suspension by way of introduction of the lipid nanoparticle solution into a controlled amount of buffer solution, or by mixing the lipid nanoparticle solution with a controlled flow rate of buffer in a second mixing region).

The processes and apparatus of the present invention further provide operational flexibility in the formulation of lipid nanoparticles and lipid nanoparticles encapsulating therapeutic agents. For example, the solubility of amphipathic lipids or lipophilic therapeutic agents is expected to decrease with addition of aqueous phase. In these instances, the concentrations of lipids or lipophilic therapeutic agents in organic fluid flow to the mixing chamber are inherently limited by the aqueous buffer concentration in said organic fluid flow, which is in turn dictated by the solvent composition desired to effect lipid nanoparticle formation. Using the four-stream MIVM, for example, amphipathic lipids or lipophilic therapeutic agents can be solubilized within individual non-aqueous organic solutions, and mixed freely against aqueous buffer to effect lipid nanoparticle formation. In this capacity, the concentrations of constituent components in the inlet streams can be maximized. In a similar capacity, the apparatus described further permits the solubilization of either non-compatible or multiple lipids or therapeutic agents in individual organic or aqueous streams.

Mixing of the fluid components can be driven using, for example, a peristaltic pump, a positive displacement pump, a pulseless gear pump, by pressurizing both the lipid-organic solution and buffer vessels or by a combination of two or more of these and/or other pump mechanisms. In one aspect, digitally controlled syringe pumps (Harvard Apparatus, PHD 2000 programmable) are used; teflon tubing (available from Upchurch Scientific) can be used for flow lines into a polypropylene or stainless steel MIVM. Lipid nanoparticles are typically formed at room temperature, but lipid nanoparticles may be formed at elevated temperatures according to the present invention. Unlike other existing approaches, there are no general requirements for buffer composition. In fact, the processes and apparatus of the present invention can formulate a lipid nanoparticle by mixing lipid in an alcohol with water. In certain aspects, the processes and apparatus of the present invention form lipid nanoparticles that are less than about 100 nm in diameter.

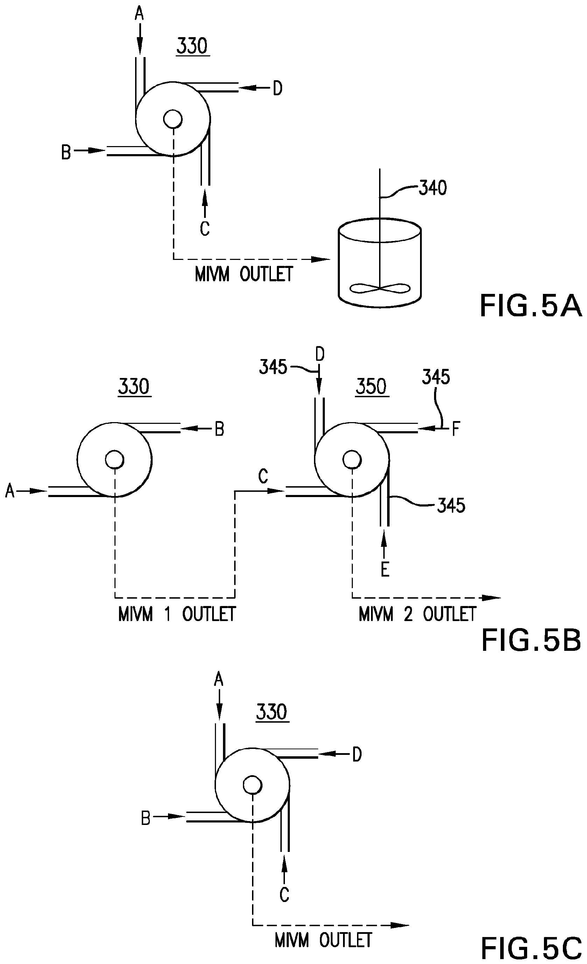

When lipid nanoparticles are prepared containing nucleic acid (such as LNPs), the ratio of nucleic acid to cationic lipid and counter ions can be optimized. For refined formulations, 70-95% nucleic acid ("NA") encapsulation after mixing, and organic solvent (e.g., ethanol) removal steps is preferred. The level of NA encapsulation is advantageously increased by immediately diluting this initial LNP formulation. Surprisingly, the processes and apparatus of the present invention provide an encapsulation efficiency, upon mixing the solutions (with therapeutic agent in one of the solution components) in the mixing environment, of up to about 90%. Three embodiments of dilution, e.g., direct dilution, are shown in FIG 5.

In the embodiment shown in FIG. 5A, the lipid nanoparticle solution formed in mixing region **330** is immediately and directly introduced into a collection vessel **340** containing a controlled amount of dilution buffer. In preferred aspects, vessel **340** includes one or more elements configured to stir the contents of vessel **340** to facilitate dilution. In one aspect, the amount of dilution buffer present in vessel **340** is substantially equal to the volume of lipid nanoparticle solution introduced thereto. As an example, lipid nanoparticle solution in 45% ethanol when introduced into vessel **340** containing an equal volume of ethanol will advantageously yield smaller particles in 22.5% ethanol.

In the embodiment shown in FIG. 5B, reservoirs **345** containing dilution buffer are fluidly coupled to a second mixing region **350**. In this embodiment, the lipid nanoparticle solution formed in mixing region **330** is immediately and directly mixed with dilution buffer in the second mixing region **350**. In certain aspects, mixing region **350** includes a MIVM arranged so that the lipid nanoparticle solution and the dilution buffer flows meet tangentially, however, connectors providing other mixing angles, such as "T"-connector, where fluid flows meet at 180° relative to each other can also be used. A pump mechanism delivers a controllable flow of buffer to mixing region **350**. In one aspect, the flow rate of dilution buffer provided to mixing region **350** is controlled to be substantially equal to the flow rate of lipid nanoparticle solution introduced thereto from mixing region **330**. This embodiment advantageously allows for more control of the flow of dilution buffer mixing with the lipid nanoparticle solution in the second mixing region **350**, and therefore also the concentration of lipid nanoparticle solution in buffer throughout the second mixing process. Such control of the dilution buffer flow rate advantageously allows for small particle size formation at reduced concentrations. See, e.g., the Examples section below.

In yet another embodiment, shown in FIG. 5C, lipid nanoparticle formation occurs coincidentally with dilution. In this instance, organic lipid and buffer solutions are introduced into the mixing environment (e.g., four-stream MIVM) at substantially non-equal flow-rates such that the resulting lipid nanoparticle solution contains a volumetric excess of dilution buffer.

In certain aspects, lipid nanoparticle producing apparatus **300** and **302** of the present invention further includes a temperature control mechanism (not shown) for controlling the temperature of the reservoirs **305** and **310.** Typically, fluid from the first reservoir **305** and the second reservoirs **310** flows into mixing chamber **330** simultaneously at separate apertures. Apparatus **300** and **302** further includes a collection reservoir **340** downstream of the mixing chamber **330** for lipid nanoparticle collection. Moreover, in certain aspects, apparatus **300** and **302** further include storage vessels upstream of any or all of the reservoirs **305, 310, 315,** and **320.** Further, any or all of the reservoirs **305**, **310**, **315,** and **320** can include jacketed stainless steel vessels equipped with an overhead mixer.

In another embodiment, the present invention provides an apparatus having an ultrafiltration system (not shown) for carrying out the processes of the present invention.

In certain aspects, apparatus includes a plurality of reservoirs and is equipped with an ultrafiltration system. An aqueous solution reservoir (not shown) and an organic solution reservoir (not shown) each have upstream preparation nanoparticles (not shown), respectively. The collection vessel (not shown) is in fluid communication with the flow ultrafiltration system. In certain aspects, ultrafiltration is used to concentrate LNP samples and then remove organic solvent (e.g., ethanol) from the formulation by buffer replacement.

In one embodiment of operation, the diluted LNP solutions are transferred to the feed reservoir of the ultrafiltration system. Concentration is performed by removing buffer and organic solution (e.g. ethanol) using, for example, cross flow cartridges 465 packed with polysulfone hollow fibers that possess internal diameters of about 0.5 mm to about 1.0 mm and about 1,000 to about 750,000 molecular weight cut-off (MWCO). The LNP are retained within the hollow fibers and re-circulated, whereas the organic solution (e.g., ethanol) and buffer components are removed from the formulation by passing through the pores of these hollow fibers. This filtrate is known as the permeate solution and is discarded. After the LNP are concentrated to the desired nucleic acid concentration, the buffer in which the LNPs are suspended may be removed by ultrafiltration and replaced by an equal volume of the final buffer. Ultrafiltration can be replaced with other methods such as conventional dialysis.

EXAMPLES

**Example 1**

This Example illustrates the use of one process of the present invention to make Lipid Nanoparticles (LNPs) which encapsulate siRNA as therapeutic product. The Example also illustrates the variation of a process parameter (e.g., inlet flow rates) according to one embodiment of the present invention.

Oligonucleotide synthesis

Oligonucleotide synthesis is well known in the art. (See US patent applications: US 2006/0083780, US 2006/0240554, US 2008/0020058, US 2009/0263407 and US 2009/0285881 and PCT patent applications: WO 2009/086558, WO2009/127060, WO2009/132131, WO2010/042877, WO2010/054384, WO2010/054401, WO2010/054405 and WO2010/054406). The siRNAs disclosed and utilized in the Examples were synthesized via standard solid phase procedures.

In one embodiment, LNPs were prepared as follows, siRNA to luciferase (See Abrams et al. Mol. Therapy (2010) 18(1):171-180; Tao et al. Mol. Therapy (2010) 18(9):1657-1666; and Morrisey et al. Nat. Biotechnology (2005) 23:1002-1007), target strand sequence ATAAGGCTATGAAGAGATA, was dissolved in citrate buffer (25 mM, 100 mM NaCl, pH 3.8) at 47$\mu$M. Lipids (CLinDMA, PEG-DMG) and cholesterol were solubilized in ethanol at a relative molar ratio of 60:38:2 (CLinDMA:Cholesterol:PEG-DMG) and a total lipid concentration of 6.7 mg/mL. The organic solution was mixed with buffer solution using a two-stream MIVM. Flow rates of inlet solution streams were varied from about 12 mL/min to 70 mL/min per stream (about 24 mL/min to 140 mL/min total). Mixing yielded a particle suspension, wherein the ethanol concentration after mixing was 50% v/v. The obtained suspension was aged at room temperature for 12-18 hours. Aliquots of aged suspension were dialyzed against phosphate buffered saline (PBS, pH 7) using a 6K-8K MWCO Spectra/Pore dialysis membrane. Dialysis was performed to exhange citrate buffer and to remove ethanol. Free (unencapsulated) siRNA does not pass through the dialysis membrane. Following dialysis, LNPs were characterized for particle size and siRNA encapsulation efficiency.

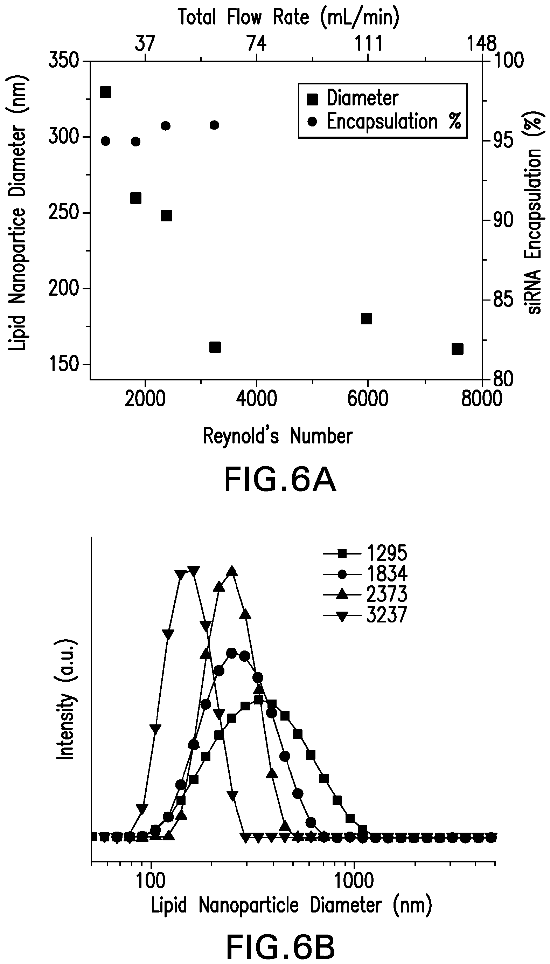

In this instance, varying the total flow rate of inlet solutions to the MIVM had a significant impact on the size of formed LNPs. At total flow rates exceeding 60 mL/min (Reynold's number of about 3200), LNPs possessed mean diameters of 150 nm +/- 20 nm (see, FIG. 6A) and narrow particle size distributions (PDI; see, FIG. 6B). Further increases in total flow rate (e.g., from about 60 mL/min to about 150 mL/min) had little impact on LNP size. At lower total flow rates, LNPs could also be prepared, although with larger particle diameters. For all flow rates examined, LNPs possessed siRNA encapsulation efficiencies greater than 95% (see, FIG. 6A). It should be appreciated that other conditions and parameters may be used and those used herein are merely exemplary.

With reference to FIG. 6A and 6B, various flow rates are modeled to show impact on Reynold's number. The Reynold's number, Re, is defined as the sum of the products of the individual stream velocities ($\mu_i$) times the characteristic length ($D_i$), divided by the sum of the kinematic viscosities ($\upsilon_i$) of the streams:

$$Re = \sum_{i=1,N} \frac{\mu_i D_i}{\upsilon_i},$$

where the characteristic dimension was taken as diameter of the MIVM mixing chamber.

**Example 2**

This Example illustrates the use of one process of the present invention to make Lipid Nanoparticles (LNPs) which encapsulate siRNA as therapeutic product. The Example also illustrates the variation of a process parameter (e.g., ethanol concentration) according to one embodiment of the present invention.

In one embodiment, LNPs were prepared as follows. siRNA to luciferase was dissolved in citrate buffer (25 mM, 100 mM NaCl, pH 3.8) at 47μM. Lipids (CLinD-MA, PEG-DMG) and cholesterol were solubilized in ethanol at a relative molar ratio of 60:38:2 (CLinDMA:Cholesterol:PEG-DMG) and a total lipid concentration of 6.7 mg/mL. The organic solution was mixed with buffer solution using either a two-stream MIVM or a four-stream MIVM. The solvent concentration (e.g., ethanol:buffer volumetric ratio) was changed by changing the feed flow rates to the MIVM. Using the two-stream MIVM, the flow rate of ethanol solution was varied between 22 mL/min and 11.8 mL/min while keeping the buffer solution flow rate constant at 22 mL/min. Mixing under these process conditions yielded particle suspensions, wherein the ethanol concentration after mixing was varied from 50% v/v to 35% v/v. To attain lower ethanol concentrations after mixing, a four-stream MIVM was used. In these instances, two additional citrate buffer (25 mM, 100 mM NaCl, pH 3.8) streams were used to reduce the ethanol concentration from 35% v/v to 10% v/v. In all cases, aliquots of LNP suspensions were diluted immediately following mixing (1:5 v/v LNP suspension to citrate buffer; 25 mM, 100 mM NaCl, pH 3.8), and dialyzed against phosphate buffered saline (PBS, pH 7) using a 6K-8K MWCO Spectra/Pore dialysis membrane. Dialysis was performed to exhange citrate buffer and to remove ethanol. Free (unencapsulated) siRNA does not pass through the dialysis membrane. Following dialysis, LNPs were characterized for particle size and siRNA encapsulation efficiency.

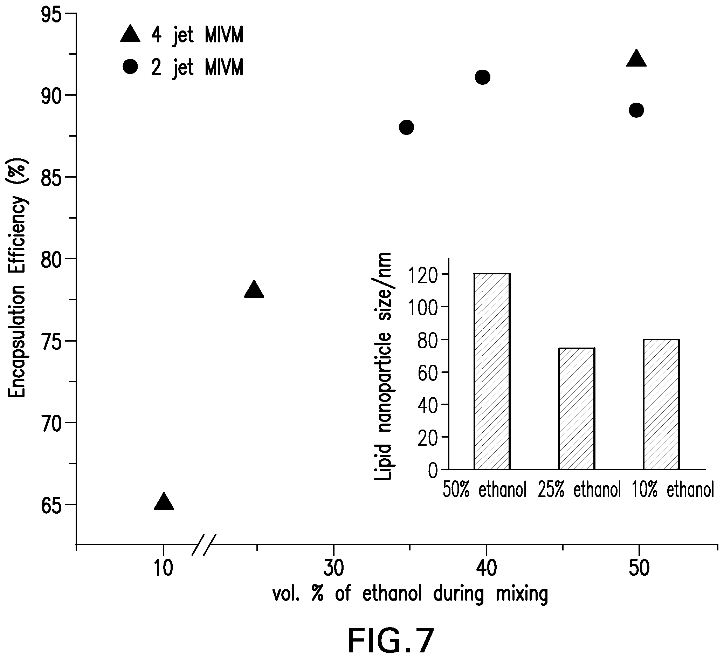

LNPs were formed under all ethanol concentrations examined in this embodiment (i.e., ranging from 10% v/v to 50% v/v). The percent ethanol at mixing was found to have a significant impact on particle size (see, FIG. 7 inset). For example, LNPs mixed in 50% v/v ethanol and diluted immediately thereafter with excess buffer, possessed a mean diameter of 120 nm. Reducing the ethanol concentration at mixing to 25% v/v, effectively reduced the mean diameter of LNPs to 70 nm. Additional lowering of ethanol concentration at mixing to 10% v/v yielded no further decreases in LNP diameters. Reductions in LNP size are attributed to increase in lipid supersaturation with decreasing ethanol content. Thus, the ethanol concentration at mixing was found to be a critical process parameter dictating LNP formation, and could thus be manipulated rationally to effect desired LNP size.

Varying the ethanol concentration at mixing according to the processes described in this embodiment additionally demonstrates the ability to effectively dilute (and stabilize) LNPs coincident with their formation (i.e. in a single mixing step). While other processes for dilution of lipsome suspensions have been described herein (see, FIG. 5A and 5B), they all require multiple steps. In the processes described in this Example, mixing of the organic lipid solution with a volumetric excess of aqueous buffer (i.e., non-equal flow rates of organic and aqueous solutions to MIVM), effectively permits the organic solution to undergo a stepwise dilution to produce a lipid nanoparticle, while simultaneously being diluted.

In certain aspects, the ethanol concentration was also found to have an impact on the encapsulation efficiency of siRNA (see, FIG. 7). Encapsulation efficiencies exceeding 87% were found for LNPs prepared with ethanol concentrations of between 35% v/v and 50% v/v at mixing. Lowering the ethanol concentration at mixing yielded LNPs with somewhat lower encapsulation efficiencies (e.g., 65% and 78% for 10% v/v and 25% v/v ethanol, respectively). It is hypothesized that at the higher ethanol concentrations, the rearrangement of lipid monomers into bilayers proceeds in a more orderly fashion compared to LNPs that are formed by dilution at lower ethanol concentrations. Without being bound by any particular theory, it is believed that these higher ethanol concentrations promote the association of nucleic acid with cationic lipids in the bilayers. In one aspect, nucleic acid encapsulation occurs within a range of ethanol concentrations between 1 0% v/v to about 50% v/v, but preferably between about 25% v/v to 35% v/v ethanol. LNP formation at 25% v/v ethanol permits optimization of both LNP size (e.g. < 150 nm, more preferably < 100 nm) and encapsulation efficiency (> 75%).

**Example 3**

This Example illustrates the use of non-alcohol organic solvents to generate LNPs according to one embodiment of the present invention.

In one embodiment, LNPs were prepared as follows. siRNA to luciferase was dissolved in citrate buffer (25 mM, 100 mM NaCl, pH 3.8) at 47μM. Lipids (CLinD-MA, PEG-DMG) and cholesterol were solubilized in tetrahydrofuran (THF) at a relative molar ratio of 60:38:2 (CLinDMA:Cholesterol:PEG-DMG) and a total lipid concentration of 6.7 mg/mL. To generate LNPs, the organic lipid solution was mixed with buffer solution using a four-stream MIVM. To obtain THF concentrations in the range of about 13% v/v to 25% v/v after mixing, two additional citrate buffer (25 mM, 100 mM NaCl, pH 3.8) solutions were used as diluent. The flow rates of organic lipid solution and siRNA-containing buffer solutions to the MIVM were varied between 22 mL/min and 30 mL/min, while the flow rate of each of the two citrate diluent streams was ranged between 44 mL/min and 120 mL/min. Aliquots of LNP suspensions were dialyzed against phosphate buffered saline (PBS, pH 7) using a 6K-8K MWCO Spectra/Pore dialysis membrane. Dialysis was performed to exchange citrate buffer and to remove ethanol. Free (unencapsulated) siRNA does not pass through the dialysis membrane. Following dialysis, LNPs were characterized for particle size and siRNA encapsulation efficiency.

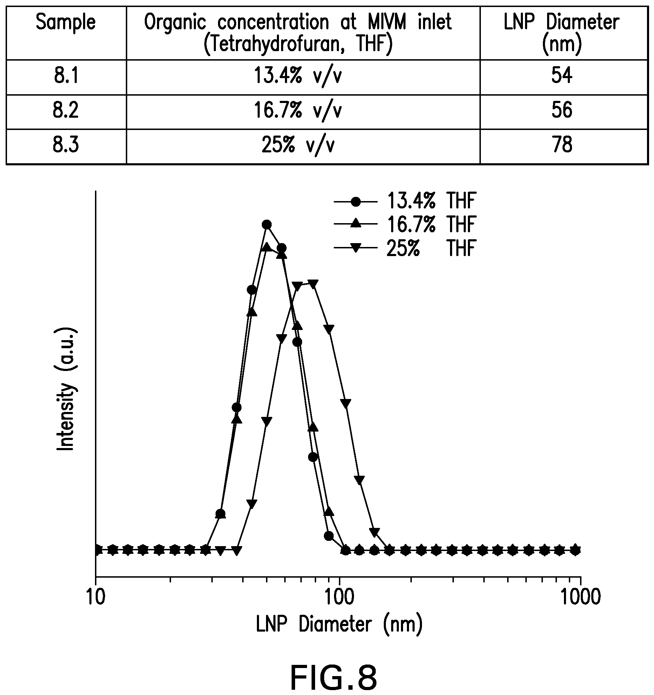

Using the processes herein described, LNPs with diameters below 80 nm, and more preferably below 55 nm, could be produced (see, FIG. 8). These LNP suspensions also possessed narrow particle size distributions (see, FIG. 8). Both reductions in LNP size and narrowing of size distributions with decreasing THF concentration are attributed to increasing lipid supersaturation under these operating conditions. Relative to formation of LNPs using ethanol (see, Example 2), the use of tetrahydrofuran for formation of LNPs permitted further reduction in particle size, especially when the organic concentration after mixing was below 25% v/v.

**Example 4**

This Example provides a comparison between processes to generate LNPs according to three embodiments of the present invention. In two embodiments, LNPs are prepared by two variations of a two-stage particle formation and dilution process, while in one embodiment a one-stage process is used.

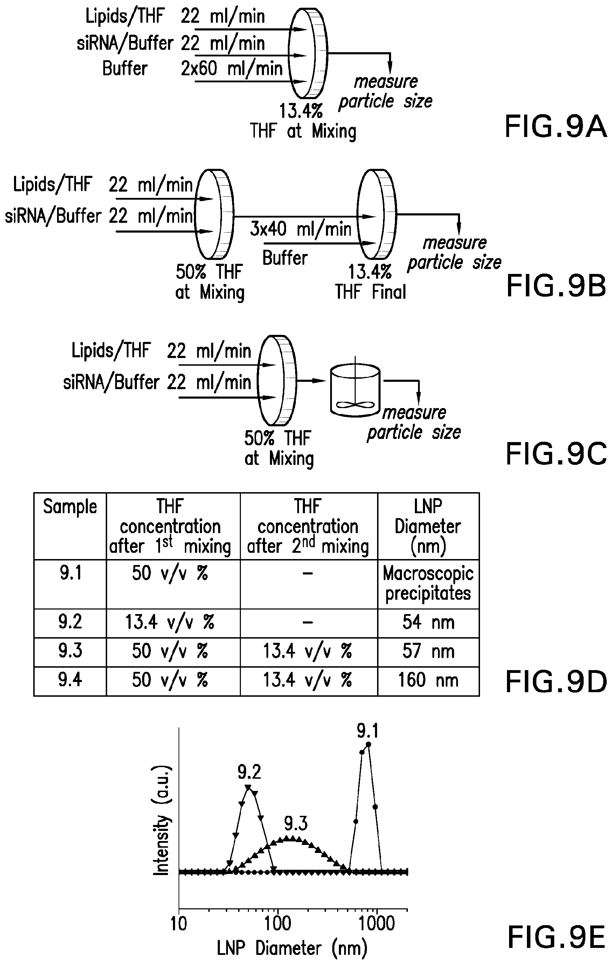

In one embodiment (e.g. one-stage process; see, FIG. 5C and FIG. 9A), LNPs were prepared as follows. siRNA to luciferase was dissolved in citrate buffer (25 mM, 100 mM NaCl, pH 3.8) at $47\mu$M. Lipids (CLinDMA, PEG-DMG) and cholesterol were solubilized in tetrahydrofuran (THF) at a relative molar ratio of 60:38:2 (CLinDMA:Cholesterol:PEG-DMG) and a total lipid concentration of 6.7 mg/mL. To generate LNPs, the organic lipid solution was mixed with buffer solutions in a four-stream MIVM. The flow rates of organic lipid solution and siRNA-containing buffer solution were 22 mL/min each, while the flow rate of each of the two citrate diluent streams was 60 mL/min. Mixing under these conditions yielded a particle suspension wherein the THF concentration after mixing was 13.4% v/v. Aliquots of LNP suspension were dialyzed against phosphate buffered saline (PBS, pH 7) using a 6K-8K MWCO Spectra/Pore dialysis membrane. Dialysis was performed to exchange citrate buffer and to remove ethanol. Free (unencapsulated) siRNA does not pass through the dialysis membrane. Following dialysis, LNPs were characterized for particle size and siRNA encapsulation efficiency.

In another embodiment (e.g. two-stage process; see, FIG. 5B and FIG. 9B), LNPs were prepared as follows. siRNA to luciferase was dissolved in citrate buffer (25 mM, 100 mM NaCl, pH 3.8) at $47\mu$M. Lipids (CLinDMA, PEG-DMG) and cholesterol were solubilized in tetrahydrofuran (THF) at a relative molar ratio of 60:38:2 (CLinDMA:Cholesterol:PEG-DMG) and a total lipid concentration of 6.7 mg/mL. To generate LNPs, the organic lipid solution was mixed with buffer solution in a first two-stream MIVM. The flow rates of organic lipid solution and siRNA-containing buffer solution were each set at 22 mL/min. Mixing under these conditions yielded a particle suspension wherein the THF concentration after mixing was 50% v/v. In a second step, the particle suspension was immediately diluted by mixing with a controlled flow rate of buffer in a second MIVM mixing chamber. In this step, particle suspension exiting the first MIVM was mixed against citrate buffer streams (25 mM, 100 mM NaCl, pH 3.8) using a four-stream MIVM. The flow rates of the particle suspension was 44 mL/min, while the total flow rate of the citrate buffer diluent was 120 mL/min. Mixing under these conditions yielded a particle suspension wherein the THF concentration after the second mixing was 13.4% v/v. Aliquots of LNP suspension were dialyzed against phosphate buffered saline (PBS, pH 7) using a 6K-8K MWCO Spectra/Pore dialysis membrane. Dialysis was performed to exchange citrate buffer and to remove ethanol. Free (unencapsulated) siRNA does not pass through the dialysis membrane. Following dialysis, LNPs were characterized for particle size and siRNA encapsulation efficiency.

In yet another embodiment (e.g. two-stage process; see, FIG. 5A), LNPs were prepared as follows. siRNA to luciferase was dissolved in citrate buffer (25 mM, 100 mM NaCl, pH 3.8) at $47\mu$M. Lipids (CLinDMA, PEG-DMG) and cholesterol were solubilized in tetrahydrofuran (THF) at a relative molar ratio of 60:38:2 (CLinDMA:Cholesterol:PEG-DMG) and a total lipid concentration of 6.7 mg/mL. To generate LNPs, the organic lipid solution was mixed with buffer solution in a two-stream MIVM. The flow rates of organic lipid solution and siRNA-containing buffer solution were each set at 22 mL/min. Mixing under these conditions yielded a particle suspension wherein the THF concentration after mixing was 50% v/v. In a second step, the particle suspension was diluted by immediate introduction of the particle suspension into a stirred reservoir containing a controlled amount of buffer solution. The reservoir contained an amount of buffer solution substantially greater than the amount of particle solution introduced thereto, such that the THF concentration after dilution was 13.4% v/v. Aliquots of LNP suspension were dialyzed against phosphate buffered saline (PBS, pH 7) using a 6K-8K MWCO Spectra/Pore dialysis membrane. Dialysis was performed to exchange citrate buffer and to remove ethanol. Free (unencapsulated) siRNA does not pass through the dialysis membrane. Following dialysis, LNPs were characterized for particle size and siRNA encapsulation efficiency.

This Example serves to compare properties of LNPs prepared according to each of the three processes described. In all intances, the organic (e.g., THF) concentration in the particle suspension after mixing and dilution was 13.4% v/v. The processes are distinguished primarily by the method by which the particle suspension after initial mixing was diluted. For example, in the one-stage process described (see, FIG. 9A), dilution occurs coincidentally with particle formation. Alternatively, in the two-stage processes (see, FIG. 9B and FIG. 9C), particle formation occurs in the first mixing step and is followed in a second step by dilution. In all instances, dilution was found to be of critical importance, as LNP suspensions in solutions of high organic content (e.g. 50% v/v THF) were unstable when dilution was not effected. Particles quickly (e.g. within one minute) grew to macroscopic sizes (see, FIGS.9D, Sample 9.1 and 9E).

Alternatively, LNPs prepared by a one-stage mixing and dilution process (see, FIG. 9A) in a four-stream MIVM were of small size (e.g. 54 nm) and possesed a narrow particle size distribution (see, FIGS. 9D, Sample 9.2 and 9E). A similar particle size was obtained when LNPs were prepared by a two-stage process in which particle formation was effected in a first MIVM mixing chamber and was followed by immediate dilution using a second MIVM mixing chamber (see, FIG. 9B). In this instance, LNPs with a mean diameter of 57 nm were formed (see, FIG. 9D, Sample 9.3), effectively demonstrating that rapid micromixing can be achieved using the apparatus of the present invention. In a final embodiment, LNPs were prepared in accordance with a two-stage process in which particle formation occurred upon mixing in a first MIVM mixing chamber and was followed by dilution in a buffer resevoir (see, FIG. 9C). In this instance, particles of large diameter (e.g. 160 nm; see, FIG. 9D, Sample 9.4) and very broad particle size distributions (see, FIG. 9E) were formed. Thus, the method by which dilution is effected is of critical importance, and processes and apparatus such as those described in this invention, act to overcome operational limitations associated with Prior Art.

## Claims

1. A process for producing lipid nanoparticles encapsulating therapeutic products, said process comprising:

   a) providing one or more aqueous solutions in one or more reservoirs;
   b) providing one or more organic solutions in one or more reservoirs, wherein one or more of the organic solutions contains a lipid and wherein one or more of the aqueous solutions and/or one or more of the organic solutions includes therapeutic products; and
   c) mixing said one or more aqueous solutions with said one or more organic solutions in a first mixing region, wherein said first mixing region is a Multi-Inlet Vortex Mixer (MIVM), wherein said one or more aqueous solutions and said one or more organic solutions are introduced tangentially into a mixing chamber within the MIVM so as to instantaneously produce a lipid nanoparticle solution containing lipid nanoparticles encapsulating therapeutic products.

2. The process of claim 1, further comprising diluting of said lipid nanoparticle solution in an aqueous buffer immediately after mixing so as to produce a diluted lipid nanoparticle solution.

3. The process of claim 2, wherein mixing said lipid nanoparticle solution in an aqueous buffer includes introducing the lipid nanoparticle solution into a buffer reservoir containing the aqueous buffer.

4. The process of claim 3, wherein the buffer reservoir contains an amount of aqueous buffer substantially equal to or greater than the amount of lipid nanoparticle solution introduced thereto.

5. The process of claim 4, wherein the buffer reservoir is stirred.

6. The process of claim 1, wherein mixing said lipid nanoparticle solution' with the aqueous buffer includes mixing in a second mixing region.

7. The process of claim 6, wherein the second mixing region is a second Multi-Inlet Vortex Mixer (MIVM) wherein the lipid nanoparticle solution and aqueous buffer are introduced tangentially into said second MIVM mixing chamber.

8. The process of claim 1, wherein said lipid nanoparticle solution has a concentration of 5% v/v to 55% v/v organic solvent.

9. The process of claim 8. wherein the diluted lipid nanoparticle solution has a concentration of less than 25% v/v organic solvent.

10. The process of claim 1, wherein said lipid nanoparticles are less than 150 nm in diameter.

11. The process of claim 1, wherein said therapeutic products are nucleic acids.

12. The process of claim 11, wherein the nucleic acids are siRNAs.

## Patentansprüche

1. Verfahren zur Herstellung von therapeutische Erzeugnisse einkapselnden Lipidnanoteilchen, wobei das Verfahren umfasst:

a) Bereitstellen einer oder mehrerer wässriger Lösungen in einem oder mehreren Reservoiren,

b) Bereitstellen einer oder mehrerer organischer Lösungen in einem oder mehreren Reservoiren, wobei eine oder mehrere der organischen Lösungen ein Lipid enthält und wobei eine oder mehrere der wässrigen Lösungen und/oder eine oder mehrere der organischen Lösungen therapeutische Erzeugnisse einschließt, und

c) Vermischen der einen oder mehreren wässrigen Lösungen mit der einen oder mehreren organischen Lösungen in einem ersten Mischungsbereich, wobei der erste Mischungsbereich ein Mehrfacheinlasswirbelmischer (MIVM) ist, wobei die eine oder mehreren wässrigen Lösungen und die eine oder mehreren organischen Lösungen tangential in eine Mischungskammer innerhalb des MIVM eingeführt werden, um so unverzüglich eine Lipidnanoteilchenlösung, welche therapeutische Erzeugnisse einkapselnde Lipidnanoteilchen enthält, herzustellen.

2. Verfahren nach Anspruch 1, weiter umfassend das Verdünnen der Lipidnanoteilchenlösung in einem wässrigen Puffer unmittelbar nach dem Vermischen, um so eine verdünnte Lipidnanoteilchenlösung herzustellen.

3. Verfahren nach Anspruch 2, wobei das Vermischen der Lipidnanoteilchenlösung in einem wässrigen Puffer das Einführen der Lipidnanoteilchenlösung in ein den wässrigen Puffer enthaltendes Pufferreservoir einschließt.

4. Verfahren nach Anspruch 3, wobei das Pufferreservoir eine Menge von wässrigem Puffer enthält, welche im Wesentlichen gleich oder größer als die darin eingeführte Menge von Lipidnanoteilchenlösung ist.

5. Verfahren nach Anspruch 4, wobei das Pufferreservoir gerührt wird.

6. Verfahren nach Anspruch 1, wobei das Vermischen der Lipidnanoteilchenlösung mit dem wässrigen Puffer das Vermischen in einem zweiten Mischungsbereich einschließt.

7. Verfahren nach Anspruch 6, wobei der zweite Mischungsbereich ein zweiter Mehrfacheinlasswirbelmischer (MIVM) ist, wobei die Lipidnanoteilchenlösung und der wässrige Puffer tangential in die zweite MIVM-Mischungskammer eingeführt werden.

8. Verfahren nach Anspruch 1, wobei die Lipidnanoteilchenlösung eine Konzentration von 5% v/v bis 55% v/v, bezogen auf organisches Lösungsmittel, aufweist.

9. Verfahren nach Anspruch 8, wobei die verdünnte Lipidnanoteilchenlösung eine Konzentration von weniger als 25% v/v, bezogen auf organisches Lösungsmittel, aufweist.

10. Verfahren nach Anspruch 1, wobei die Lipidnanoteilchen weniger als 150 nm im Durchmesser betragen.

11. Verfahren nach Anspruch 1, wobei die therapeutischen Erzeugnisse Nukleinsäuren sind.

12. Verfahren nach Anspruch 11, wobei die Nukleinsäuren siRNAs sind.

**Revendications**

1. Procédé pour la production de nanoparticules lipidiques encapsulant des produits thérapeutiques, ledit procédé comprenant :

a) la fourniture d'une ou de plusieurs solutions aqueuses dans un ou plusieurs réservoirs ;

b) la fourniture d'une ou de plusieurs solutions organiques dans un ou plusieurs réservoirs, dans lequel une ou plusieurs des solutions organiques contiennent un lipide et dans lequel une ou plusieurs des solutions aqueuses et/ou une ou plusieurs des solutions organiques comprennent des produits thérapeutiques ; et

c) le mélange d'une ou de plusieurs solutions aqueuses avec lesdites une ou plusieurs solutions organiques dans une première région de mélange, dans lequel ladite première région de mélange est un mélangeur vortex à entrées multiples (MIVM), dans lequel lesdites une ou plusieurs solutions aqueuses et lesdites une ou plusieurs solutions organiques sont introduites tangentiellement dans une chambre de mélange au sein du MIVM de façon à produire instantanément une solution de nanoparticules lipidiques contenant des nanoparticules lipidiques encapsulant des produits thérapeutiques.

2. Procédé selon la revendication 1, comprenant en outre la dilution de ladite solution de nanoparticules lipidiques dans un tampon aqueux immédiatement après le mélange afin de produire une solution diluée de nanoparticules lipidiques.

3. Procédé selon la revendication 2, dans lequel le mélange de ladite solution de nanoparticules lipidiques dans un tampon aqueux comprend l'introduction de la solution de nanoparticules lipidiques dans un réservoir de tampon contenant le tampon aqueux.

4. Procédé selon la revendication 3, dans lequel le réservoir de tampon contient une quantité de tampon aqueux sensiblement égale ou supérieure à la quantité de solution de nanoparticules lipidiques introduite à l'intérieur.

5. Procédé selon la revendication 4, dans lequel le réservoir de tampon est agité.

6. Procédé selon la revendication 1, dans lequel le mélange de ladite solution de nanoparticules lipidiques avec le tampon aqueux inclut le mélange dans une seconde région de mélange.

7. Procédé selon la revendication 6, dans lequel la seconde région de mélange est un second mélangeur vortex à entrées multiples (MIVM) dans lequel la solution de nanoparticules lipidiques et le tampon aqueux sont introduits tangentiellement dans ladite seconde chambre de mélange du MIVM.

8. Procédé selon la revendication 1, dans lequel ladite solution de nanoparticules lipidiques a une concentration de 5 % v/v à 55 % v/v de solvant organique.

9. Procédé selon la revendication 8, dans lequel la solution diluée de nanoparticules lipidiques a une concentration inférieure à 25 % v/v de solvant organique.

10. Procédé selon la revendication 1, dans lequel lesdites nanoparticules lipidiques ont un diamètre inférieur à 150 nm.

11. Procédé selon la revendication 1, dans lequel lesdits produits thérapeutiques sont des acides nucléiques.

12. Procédé selon la revendication 11, dans lequel les acides nucléiques sont des pARNi.

100

FIG. 1

## Two-stage liposome formation process

FIG.2A

## One-stage liposome formation process

FIG.2B

Two-stream  Multi-Inlet  Vortex  Mixer

300

FIG.3A

Four-stream  Multi-Inlet  Vortex  Mixer

302

FIG.3B

FIG.4A

330

FIG.4B

FIG.5A

FIG.5B

FIG.5C

FIG.6A

FIG.6B

FIG.7

EP 2 558 074 B1

| Sample | Organic concentration at MIVM inlet (Tetrahydrofuran, THF) | LNP Diameter (nm) |
|--------|------------------------------------------------------------|-------------------|
| 8.1 | 13.4% v/v | 54 |
| 8.2 | 16.7% v/v | 56 |
| 8.3 | 25% v/v | 78 |

FIG.8

Lipids/THF  22  ml/min
siRNA/Buffer 22  ml/min
Buffer      2x60  ml/min

*measure particle size*

13.4%
THF at Mixing

**FIG.9A**

Lipids/THF  22  ml/min
siRNA/Buffer 22  ml/min

3x40  ml/min
Buffer

*measure particle size*

50% THF
at Mixing

13.4%
THF Final

**FIG.9B**

Lipids/THF  22  ml/min
siRNA/Buffer 22  ml/min

*measure particle size*

50% THF
at Mixing

**FIG.9C**

| Sample | THF concentration after 1st mixing | THF concentration after 2nd mixing | LNP Diameter (nm) |
|--------|-----------------------------------|-----------------------------------|--------------------|
| 9.1 | 50 v/v % | – | Macroscopic precipitates |
| 9.2 | 13.4 v/v % | – | 54 nm |
| 9.3 | 50 v/v % | 13.4 v/v % | 57 nm |
| 9.4 | 50 v/v % | 13.4 v/v % | 160 nm |

**FIG.9D**

**FIG.9E**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5478860 A, Wheeler
- WO 0105373 A, Knopov
- US 20040142025 A
- WO 2009061406 A
- WO 2009126933 A
- WO 2009132131 A1
- WO 2010042877 A
- WO 2010146740 A
- WO 2010105209 A
- WO 05026372 A
- US 20030077829 A
- US 2005008689 A
- US 5885613 A
- US 20060083780 A
- US 20060240554 A
- US 20080020058 A
- US 20090263407 A
- US 20090285881 A
- WO 2009086558 A
- WO 2009127060 A
- WO 2009132131 A
- WO 2010054384 A
- WO 2010054401 A
- WO 2010054405 A
- WO 2010054406 A

### Non-patent literature cited in the description

- Thug Transport in antimicrobial and anticancer chemotherapy. CRC Press, 1995
- LIU Y. et al. *Chemical Engineering Science,* 2008, vol. 63, 2829-2842
- MARIAN E. GINDY et al. Composite Block Copolymer Stabilized Nanoparticles: Simultaneous Encapsulation of Organic Actives and Inorganic Nanostructures. *Langmuir,* 01 January 2008, vol. 24 (1), 83-90
- ABRAMS. *Molecular Therapy,* 2010, vol. 18 (1), 171
- ABRAMS et al. *Mol. Therapy,* 2010, vol. 18 (1), 171-180
- TAO et al. *Mol. Therapy,* 2010, vol. 18 (9), 1657-1666
- MORRISEY et al. *Nat. Biotechnology,* 2005, vol. 23, 1002-1007